(19) Europäisches Patentamt
European Patent Office
Office européen des brevets

(11) EP 2 485 118 A1

(12) EUROPEAN PATENT APPLICATION
published in accordance with Art. 153(4) EPC

(43) Date of publication:
08.08.2012 Bulletin 2012/32

(51) Int Cl.:
*G06F 3/01* (2006.01)          *G06K 9/00* (2006.01)
*A61B 3/113* (2006.01)         *G06F 3/033* (2006.01)
*G06K 9/20* (2006.01)

(21) Application number: 09849935.3

(22) Date of filing: 29.09.2009

(86) International application number:
PCT/CN2009/001105

(87) International publication number:
WO 2011/038527 (07.04.2011 Gazette 2011/14)

(84) Designated Contracting States:
AT BE BG CH CY CZ DE DK EE ES FI FR GB GR
HR HU IE IS IT LI LT LU LV MC MK MT NL NO PL
PT RO SE SI SK SM TR

(71) Applicant: Alcatel Lucent
75007 Paris (FR)

(72) Inventor: ZHUANG, Longpeng
Beijing 100102 (CN)

(74) Representative: Michalski, Stefan
Michalski Hüttermann & Partner
Patentanwälte
Neuer Zollhof 2
40221 Düsseldorf (DE)

(54) **METHOD FOR VIEWING POINTS DETECTING AND APPARATUS THEREOF**

(57) The present invention provides an apparatus for detecting a fixation point based on face detection and image measurement, comprising: a camera for capturing a face image of a user; a reference table acquiring unit for acquiring a reference table comprising relations between reference face images and line-of-sight directions of the user; and a calculating unit for performing image measurement based on the face image of the user captured by the camera and looking up the reference table in a reference table acquiring unit, so as to calculate the fixation point of the user on the screen.

The present invention further provides a method of detecting a fixation point based on face detection and image measurement.

The present invention can detect a line-of-sight of the user, which provides great convenience for movement of a cursor.

Apparatus 100 for Detecting a Fixation Point

Fig.1

**Description**

FIELD OF THE INVENTION

The embodiments of the present invention relate to the field of image processing, and specifically relate to a method and apparatus for detecting a fixation point based on face detection and image measurement.

DESCRIPTION OF THE RELATED ART

With the evolution of the image processing technology, when a user desires to move a cursor from an area to another area on the screen of a current video display (for example, a screen of a desktop or laptop, a screen of a TV, etc.), the user usually needs to leverage an auxiliary device (for example, a mouse or a touchpad or a remote controller) to perform the action. However, for some users, movement of hands is restricted due to some reasons, for example, physiological handicap or being injured; thus it would be difficult or even impossible to move the cursor. Additionally, even if the hands move normally, in some special scenarios, it is desirable to perform the cursor movement without using hand or shorten the movement distance of the hand to the least.

Further, even in case of not moving the cursor, some applications may need to detect a fixation point of a user on the screen so as to perform subsequent processing and operation.

Nowadays, with the growing popularization of camera and the increasing emergence of mature face detection algorithms, it is feasible for detecting a video-image based on the camera. Thus, it is desirable for a technique of detecting a fixation point utilizing a camera, so as to detect a fixation point of a user on a screen.

SUMMARY OF THE INVENTION

According to one aspect of the present invention, an apparatus for detecting a fixation point is provided, which is used for calculating a fixation point of a user on a screen, comprising: a camera for capturing a face image of the user; a reference table acquiring unit for acquiring a reference table comprising relations between reference face images and line-of-sight directions of the user; and a calculating unit for performing image measurement based on the face image of the user captured by the camera, and looking up the reference table in the reference table acquiring unit, to calculate the fixation point of the user on the screen.

Preferably, the reference table acquiring unit comprises at least one of the following: a reference table constructing unit for constructing the reference table based on at least one reference face image of the user captured by the camera; and a reference table storing unit that stores the reference table which has already been constructed.

Preferably, the calculating unit comprises: a line-of-sight direction calculating unit for measuring a distance between a middle point of two pupils of the user in the face image of the user and the camera based on a location of the camera and calculating the line-of-sight direction of the user through looking up the reference table; and a fixation point calculating unit for calculating the fixation point of the user on the screen based on the location of the camera, the distance between the middle point of two pupils of the user and the camera, and the line-of-sight direction of the user.

Preferably, the apparatus for detecting a fixation point further comprises: a cursor moving unit, wherein, after the fixation point is calculated, if the fixation point is located within the screen, then the cursor moving unit moves the cursor on the screen to the fixation point.

Preferably, if the distance between the fixation point and the current cursor is less than a predefined value, then the cursor moving unit does not move the cursor.

Preferably, the apparatus for detecting a fixation point further comprises: an auxiliary unit for performing operation at the cursor location. Preferably, the auxiliary unit comprises at least one of a mouse, a keyboard, a touchpad, a handle, and a remote controller.

According to another aspect of the present invention, a method of detecting a fixation point is provided, for calculating a fixation point of a user on a screen, which comprises the following steps: a reference table acquiring step of acquiring a reference table comprising relations between reference face images and line-of-sight directions of the user; a fixation point calculation step of capturing the face image of the user using a camera and performing image measurement and looking up the reference table, to calculate the fixation point of the user on the screen.

Preferably, the reference table acquiring step comprises: using the camera to acquire at least one reference face image of the user to construct the reference table comprising relations between the reference face images and the line-of-sight directions of the user; or directly obtaining the reference table which has already been constructed.

Preferably, the fixation point calculating step comprises: measuring a distance between a middle point of two pupils of the user in the face image of the user and the camera based on a location of the camera, and calculating the line-of-sight direction of the user through looking up the reference table; and calculating the fixation point of the user on the screen based on the location of the camera, the distance between the middle point of two pupils of the user and the camera, and the line-of sight direction of the user.

Preferably, the method of detecting a fixation point further comprises: after the fixation point is calculated, if the fixation point is located within the screen, then moving the cursor on the screen to the fixation point.

Preferably, if the distance between the fixation point and the current cursor is less than a predefined value, then the cursor is not moved. Preferably, the predefined value can be set as required.

According to a further aspect of the present invention, a multi-screen computer is provided, which have multiple screens around a user, wherein the multi-screen computer comprises the apparatus for detecting a fixation point according to the present invention.

BRIEF DESCRIPTION OF THE DRAWINGS

The above and other features of the present invention will become more apparent through the following description with reference to the accompanying drawings, wherein:

Fig. 1 is a block diagram of an embodiment of an apparatus for detecting a fixation point according to the present invention;

Fig. 2a is a flow chart of an embodiment of a method of detecting a fixation point according to the present invention;

Fig. 2b is a flow chart of a sub-step of the method of detecting a fixation point in Fig. 2a;

Fig. 3 is a diagram of a reference face image in an exemplary coordinate system;

Fig. 4 is a diagram of an exemplary face image;

Fig. 5a is a diagram of different face directions;

Fig. 5b is a coded map of different face directions;

Fig. 6a is a diagram of an eyeball model in different directions;

Fig. 6b is a diagram of a relation between a vertical angle and a horizontal angle of the eyeball model in the exemplary coordinate system;

Fig. 7 is a diagram of a relation between a projection round radius and a cone vertex angel;

Fig. 8 is a diagram of an angle between the projection ($A_n$' B') of a connection line between a camera and a user and the X axis ($A_0$' C');

Fig. 9 is a principle diagram of detecting a fixation point according to the present invention;

Fig. 10 is a block diagram of an example of an eyeball direction table; and

Fig. 11 is a block diagram of an example of a projection round radius - cone vertex angle table.

DETAILED DESCRIPTION OF THE INVENTION

Hereinafter, the principle and implementation of the present invention will become more apparent through the description on the embodiments of the present invention with reference to the accompanying drawings. It should be noted that the present invention should not be limited to the specific embodiments as described below.

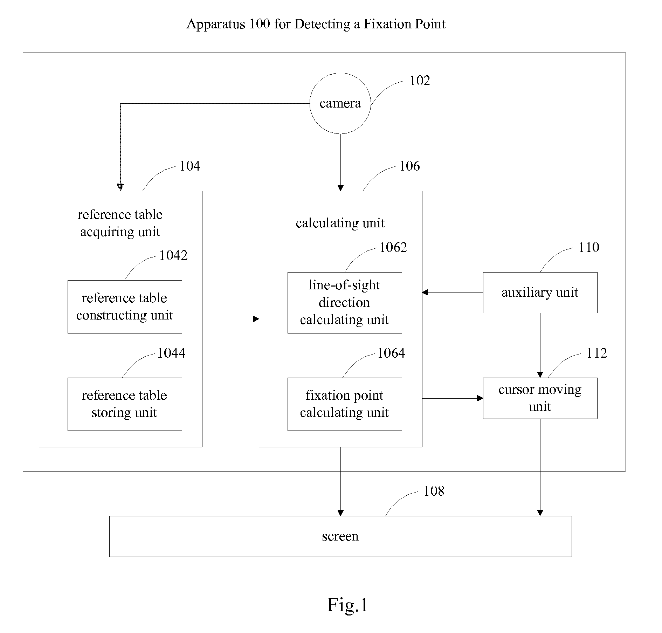

Fig. 1 is a block diagram of an embodiment of an apparatus 100 for detecting a fixation point according to the present invention.

As illustrated in Fig. 1, the apparatus 100 for detecting a fixation point comprises a camera 102, a reference table acquiring unit 104, and a calculating unit 106. The camera 102 can be a common camera in the art, for capturing a face image of a user. The reference table acquiring unit 104 is for acquiring a reference table comprising relations between reference face images and line-of-sight directions of the user. The calculating unit 106 can calculate the line-of-sight direction of the user through the reference table and then calculate the fixation point of the user on a screen 108.

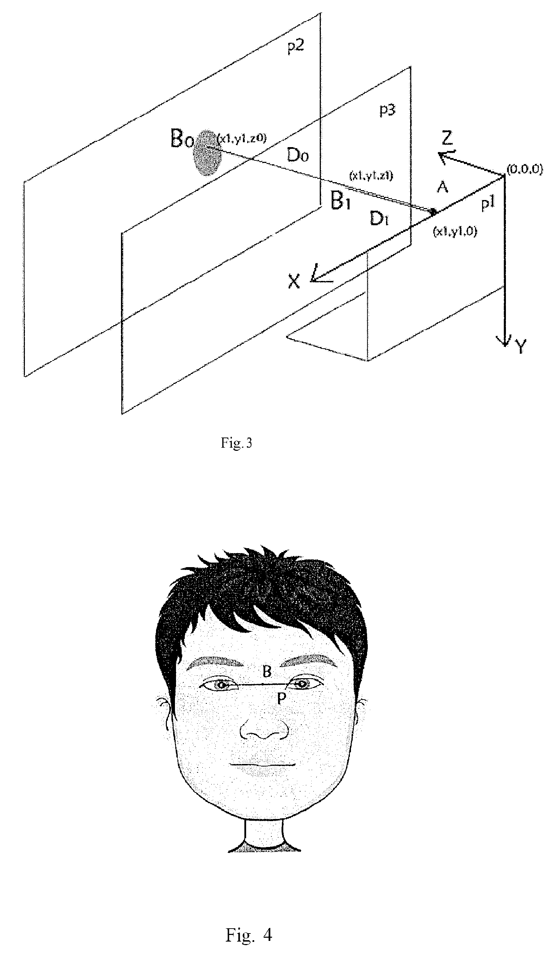

Hereinafter, as an example, a specific implementation of the reference face image and the reference table, as well as the operation of each component in the apparatus 100 for detecting a fixation point, is illustrated with reference to Figs. 3-9.

In order to perform locating and calculation, a 3-axis coordinate system as illustrated in Fig. 3 can be established, where the origin of the coordinate system is located at the upper left corner of the screen. From the perspective of a computer user, the axis extending from left to right along an upper edge of the screen is X axis, the axis extending from top to down along a left edge of the screen is Y axis, while the axis extending from far (screen end) to near (user end) vertical to the screen is Z axis. The camera 102 is installed at point A with a coordinate ($x_1$, $y_1$, 0). As illustrated in Fig. 4, point B is a middle point between two pupils of the user. The AB distance is the distance between point A (the location of the camera) and point B. Pupil distance is the distance between centers of the two pupils of the user in the image.

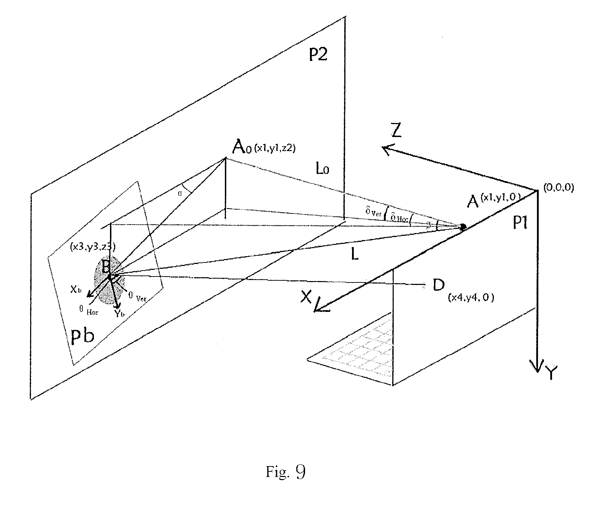

For example, suppose the screen is in plane 1 (P1), while the front face of the camera 102 is parallel to the plane 1. And, suppose point B is located in a plane 2 (P2) or plane 3 (P3) parallel to plane 1. As illustrated in Fig. 9, the plane Pb refers to the plane where point B is located and vertical to the straight line AB. In the plane Pb, Yb axis is a cross line between the vertical plane where the straight line AB is located and the plane Pb, and Xb axis is a straight line within the plane Pb and vertical to the Yb axis.

According to the principle of "the farther, the smaller; the nearer, the greater," the distance between point A and B can be detected based on the size of the face image or relevant component distance. In order to perform the measurement, a reference face image is introduced. As illustrated in Fig. 3, the reference face image refers to the image captured by the camera when the face of the user right ahead of the camera and the distance between A and B is Do (the distance between the camera and the middle point of the two pupils). Due to possible existence of relative error, more number of reference images can reduce the relative error and result in a more accurate detection result. For example, two reference face images are introduced, with one having an AB distance of Do and the other having a shorter AB distance of $D_1$. In order to obtain the reference face images, the camera 102 should be setted at point A with a coordinate $(x_1, y_1, 0)$ in the coordinate system, and the user should be located in a suitable location so as to guarantee that point B (the middle point between two eyes, as illustrated in Fig. 4) is located at $(x_1, y_1, z_0)$ or $(x_1, y_1, z_1)$ in the coordinate system, and $(x_1, y_1, z_0)$ or $(x_1, y_1, z_1)$ should meet the following equations:

$$z_0 - 0 = D_0 \qquad\qquad (1)$$

$$z_1 - 0 = D_1 \qquad\qquad (2)$$

When the user face is detected using a face detection/identification algorithm, the center of each pupil can be located such that the point B and distance between the centers of the two pupils can be obtained, as illustrated in Fig. 4. If the face image of the user is a reference face image with a distance of Do, then the distance between the centers of the two pupils is the reference pupil distance $P_0$. If the face image of the user is a reference face image with a distance of $D_1$, then the distance between the centers of the two pupils is the reference pupil distance $P_1$.

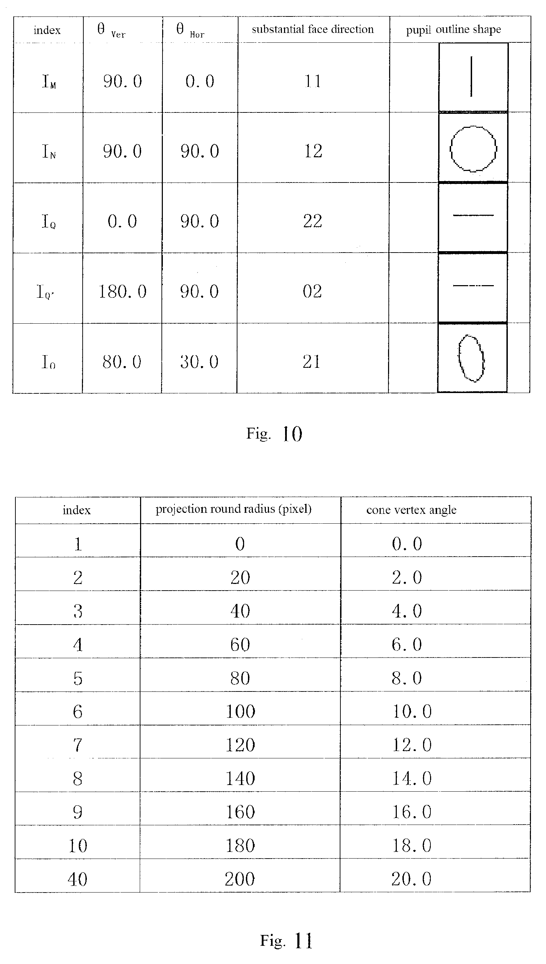

In this embodiment, the reference table comprises an eyeball direction table and a projection round radius - cone vertex angle table, which will be described in detail hereinafter with reference to Figs. 10 and 11.

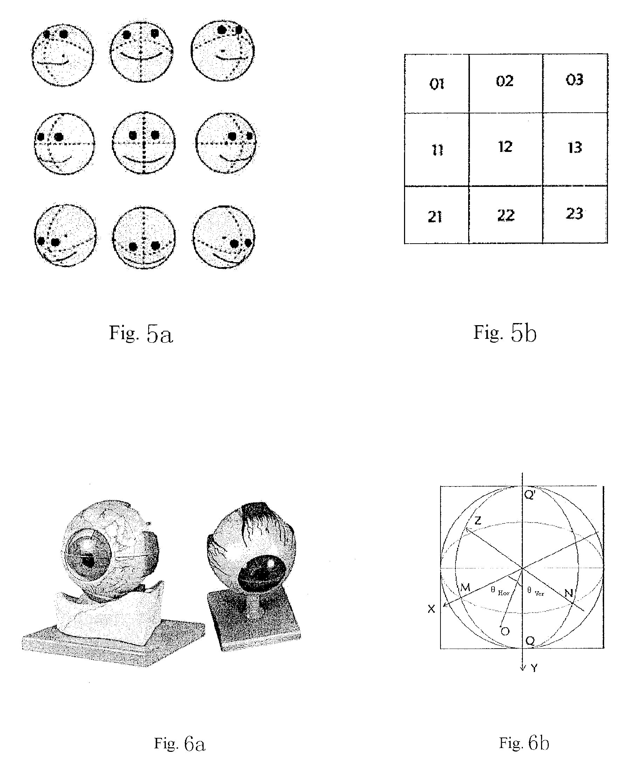

When the user looks towards different areas in the screen, the user may turn the head such that the face directly (or almost directly) faces the area. Fig. 5a illustrates possible face directions. Based on different orientations of the face, the face orientations can be substantially divided into 9 directions herein, and different face directions are coded, with the specific codes illustrated in Fig. 5b.

When capturing the face of the user, outlines of pupils of the user's eyes can be determined simultaneously. In the present embodiment, a user's eye can be regarded as a sphere, while a pupil can be regarded as a circle on the surface of the eyeball. Moreover, a pupil can directly face towards a fixation point on the screen. Fig. 6a illustrates an eyeball model having two different eyeball directions. As illustrated in Fig. 6a, when the user looks towards different directions, the pupils will change directions with the eyes. In the image captured by the camera, the outlines of the pupils will change from one kind of oval shape to another kind of oval shape. Based on the outlines of the pupils and the face directions, the rotation angle of each eyeball can be obtained, comprising:

The vertical rotation angle of the left eyeball: $^\theta$Ver-L,

the horizontal rotation angle of the left eyeball: $^\theta$Hor-L,

the vertical rotation angle of the right eyeball: $^\theta$Ver-R,

the horizontal rotation angle of the right eyeball: $^\theta$Hor-R.

The $^\theta$Ver herein refers to the angle between the pupil direction and the Yb axis, while $^\theta$Hor refers to the angle between the pupil direction and the Xb axis. In order to enhance the performance of calculation in the eyeball direction, so as to obtain the above 4 angles, i.e., $^\theta$Ver-L, $^\theta$Hor-L, $^\theta$Ver-R, and $^\theta$Hor-R, an eyeball direction table is introduced to list all possible eyeball directions and their rotation angles. With reference to Fig. 10, the table at least comprises the following 5 columns of information: the first column represents index; the second column represents the vertical rotation angle $^\theta$Ver; the third column represents the horizontal rotation angle $^\theta$Hor, the fourth column represents a corresponding substantial face direction; and the fifth column comprises images related to pupil outlines after the eyes (pupils) rotated vertically and horizontally. The values in the second column ($^\theta$Ver) and the third column ($^\theta$Hor) vary between 0.0°- 180.0°. As illustrated in Fig. 6b, the values of $^\theta$Ver and $^\theta$Hor must satisfy that the point 0 is located on the sphere surface. The value ranges of the eyeball direction table are $^\theta$Ver and $^\theta$Hor corresponding to sampling points at the side of the sphere surface facing the camera (i.e., the negative axis direction of Z axis), and the outline shapes of the pupils at the sampling points viewed by the camera. The more intense the sampling points are, the less are the increments of $\theta$Ver and $\theta$Hor, and the more accurate are the results, but the larger is the load to be performed. The default angle increment is 0.1°. As an example, Fig. 10 merely illustrates the table contents when the pupils are at point M, point N, point Q, and point Q' (wherein the index column should be gradually incremented by an integer value in actual implementation, such as 1,2, 3, etc., and here for the convenience of expression, they are written as $I_M$, $I_N$, $I_Q$, etc.).

The use process of this table is specified below: after obtaining the image of eyes, the outline of the left eye (or right eye) is extracted to find a most suited outline in the table, thereby obtaining the following angles: $\theta$Ver-L, $\theta$Hor-L, (or $\theta$Ver-R, $\theta$Hor-R) From the table, we can see that the points symmetrical around the sphere central point in Fig. 6, for example, points Q and Q', are identical to the pupil outlines viewed by the camera, which needs judgment through the face direction. In the actual operation process, interpolation of the ranges of the user's possible angles $\theta_{Ver}$ and $\theta$Hor can be densified, based on the location relation of the user relative to the camera 102 and the size of the screen, which helps to improve the accuracy of the results.

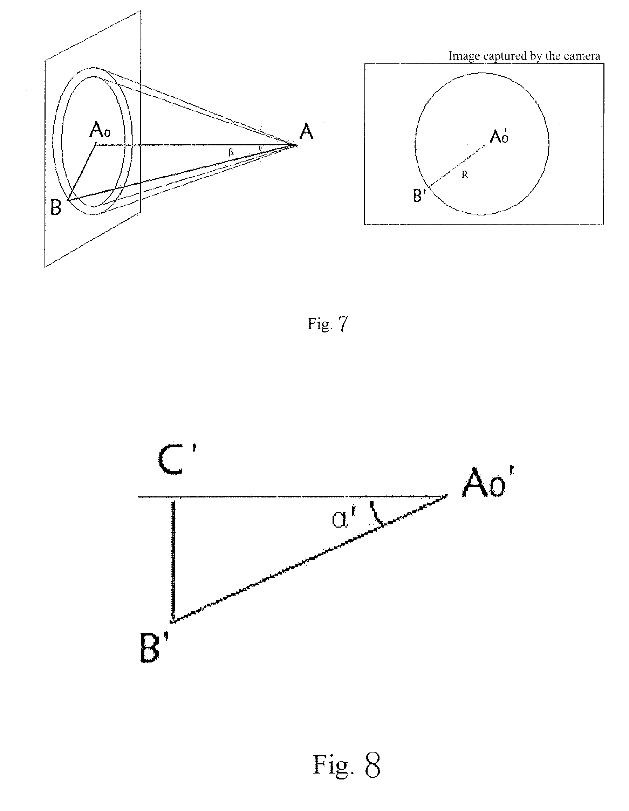

For the camera 102, all points on a cone side surface will be projected onto a circle in the image captured by the camera. Thus, once the radius of the circle in the image captured by the camera is obtained, the vertex angle of the cone can be determined, as illustrated in Fig. 7. In order to better describe the vertex angle of the cone, Fig. 11 illustrates relations between all possible vertex angles of the cone and the radius of the projection round for a certain camera. The distance unit in the table is pixel, which can be converted into other units. The range of the radius values of the projection round is 0 - $R_{MAX}$. $R_{MAX}$ is the farthest distance from the image center to a corner of the image. The contents in the table can be set based on different cameras, because different cameras have different resolutions, focal distances, and wide-angles. The suggested granularity of increment of the projection round radius is 5 pixels. The smaller the granularity is, the more accurate are the results, but the more times of calculation and comparison are required when being executed. As an example, the projection round radius - cone vertex table as illustrated in Fig. 11 adopts a unit of 10 pixels, with the $R_{MAX}$ of the camera is 200 pixels, the maximum viewing angle of the camera is 40° (20° for left and right, respectively).

In actual implementation process, the interpolation of the angle corresponding to the location where the user is always located (i.e., the vertex angle of the cone) is densified, based on the location relation of the user relative to the camera 102, which helps to improve the accuracy of the results.

In the present embodiment, the reference table acquiring unit 104 comprises a reference table constructing unit 1042 which constructs the above mentioned eyeball direction table and projection round radius-cone vertex table utilizing the reference face images having distances Do and $D_1$ captured by the camera 102. Additionally, the reference table acquiring unit 104 further comprises a reference table storing unit 1044. If the reference table has been constructed and stored in the reference table storing unit 1044, then the reference table acquiring unit 104 can directly read it therefrom. Moreover, the reference table constructed by the reference table constructing unit 1042 can be stored into the reference table storing unit 1044.

The calculating unit 106 can comprise a line-of-sight direction calculating unit 1062 and a fixation point calculating unit 1064. Herein, the line-of-sight direction calculating unit 1062 measures the distance from the middle point of two pupils of the user in the user's face image to the camera based on the location of the camera, and calculates the line-of sight direction of the user through looking up the reference table. Specifically, the line-of-sight direction calculating unit 1062 adopts a mature face detection/identification algorithm, for example, OpenCV, to detect the substantial direction of the user face, the outlines of the user's eyes and pupils, and the pupil distance P. The AB distance L is calculated using the pupil distance P, reference pupil distances $P_0$ and $P_1$. The distance and image size have the following relations:

$$\text{Distance} \times \text{image size} \approx \text{constant} \qquad (3)$$

Therefore, the AB distance L and pupil distance P meet the following equations:

$$L \times P \approx D_0 \times P_0 \qquad (4)$$

$$L \times P \approx D_1 \times P_1 \qquad (5)$$

In order to improve the accuracy of the results, the equations (4) and (5) are combined to obtain:

$$L = (P_0 \times D_0/P + P_1 \times D_1/P)/2 \qquad (6)$$

The line-of-sight direction calculating unit 1062 further calculates angle $\alpha$ and $\beta$. Specifically, $\alpha$ refers to the angle between the middle line $A_0B$ in plane 2 and X axis, wherein $A_0$ is a vertical projection point of point A on the plane P2, point B is the middle point between two pupils (as illustrated in Fig. 9). Because plane 2 is parallel to plane 1, the angle $\alpha$ is identical to the projection angle $\alpha'$ in the image captured by the camera.

$$\alpha = \alpha' \qquad (7)$$

Fig. 8 illustrates points $A_0'$, B' and angle $\alpha'$ within the image, and they satisfy:

$$A_0'B' \times \sin(\alpha') = B'C' \qquad (8)$$

$A_0'B'$ and B'C' indicate the lengths between these points in the image. Thus, the value of the angle $\alpha'$ is:

$$\alpha' = \arcsin(B'C'/A_0'B') \qquad (9)$$

After obtaining the length of $A_0'B'$ in the image captured by the camera, the line-of-sight direction calculating unit 1062 can search in the projection round radius-cone vertex angle table to find the most suitable row in which the projection round radius value matches the length $A_0'B'$. In this way, the cone vertex angle in the same row is the angle $\beta$. Then, the line-of-sight direction calculating unit 1062 calculates the coordinate of point B. By utilizing the previously obtained result, when point B is located at the lower left to the point $A_0$ (viewing the image angle from the front, as illustrated in Fig. 9; the same below), the coordinate $(x_3, y_3, z_3)$ of point B can be calculated in accordance with the following equations:

$$x_3 = x_1 + L \times \sin(\beta) \times \cos(\alpha) \qquad (10)$$

$$y_3 = y_1 + L \times \sin(\beta) \times \sin(\alpha) \qquad (11)$$

$$z_3 = z_2 = L \times \cos(\beta) \qquad (12)$$

When point B is located right to point $A_0$ (including upper right, lower right), the sign for addition in equation (10) is changed to be the sign for minus; and when point B is located above the point $A_0$ (including upper left, upper right), the sign for addition in equation (11) is changed to be the sign for minus.
Next, the line-of-sight direction calculating unit 1062 calculates the rotation angel of the eyeballs. Specifically, based on the image captured by the camera, the outline of the pupil of the left eye is detected to find a most suitable outline from the above mentioned eyeball direction table, and further, in combination with the face direction, to thereby obtain the vertical rotation angle $\theta$Ver-L of the eyeball relative to the Yb axis and the horizontal rotation angle $\theta$Hor-L relative to the Xb axis. $\theta$Ver-R and $\theta$Her-R of the right eye can also be obtained in accordance with the same steps.
Then, the line-of-sight direction calculating unit 1062 calculates the line-of-sight direction of the user:

$$\theta_{Ver} = (\theta_{Ver-L} + \theta_{Ver-R})/2 \qquad (13)$$

$$\theta_{Hor} = (\theta_{Hor-L} + \theta_{Hor-R})/2 \qquad (14)$$

The above line-of-sight direction is relative to the Xb axis and Yb axis in the plane Pb, which should be further converted into the angle relative to the X axis and Y axis. Therefore, the line-of-sight direction calculating unit 1062 calculates the angle $\delta_{Hor}$ between the horizontal axis Xb of the plane Pb and the horizontal axis X axis of the plane P1 and the angle $\delta_{Ver}$ between the Yb axis and the vertical axis Y axis of the plane P1, as illustrated in Fig. 9, and they satisfy:

$$\tan(\delta_{Hor})=[L\times \sin(\beta)\times \cos(\alpha)]/[L\times \cos(\beta)] \qquad (15)$$

$$\tan(\delta_{Ver})=[L\times \sin(\beta)\times \sin(\alpha)]/[L\times \cos(\beta)] \qquad (16)$$

Thereby, $\delta_{Hor}$ and $\delta_{Ver}$ can be obtained:

$$\delta_{Hor}=\arctan\{L\times \sin(\beta)\times \cos(\alpha)/[L\times \cos(\beta)]\} \qquad (17)$$

$$\delta_{Ver}=\arctan\{L\times \sin(\beta)\times \sin(\alpha)/[L\times \cos(\beta)]\} \qquad (18)$$

In combination with the previously obtained $\theta_{Ver}$ and $\theta_{Hor}$, the line-of-sight direction calculating unit 1062 can work out the final $\theta_{Ver-Final}$ and $\theta_{Hor-Final}$:

$$\theta_{Ver-Final} = \theta_{Ver} + \delta_{Ver} \qquad (19)$$

$$\theta_{Hor-Final} = \theta_{Hor} + \delta_{Hor} \qquad (20)$$

Afterwards, the fixation point calculating unit 1064 calculates the fixation point of the user on the screen 108 based on the location of the camera, the distance from the middle point between two pupils of the user to the camera, and the line-of-sight direction of the user. Specifically, the fixation point calculating unit 1064 calculates the coordinate $(x_4, y_4, 0)$ of the fixation point D on the screen 108 in accordance with the following equation based on $\theta_{Ver-Final}$ and $\theta_{Hor-Final}$ calculated by the line-of-sight direction calculating unit 1062:

$$L_0=L\times\cos (\beta) \qquad (21)$$

$$x_4=L_0/\tan(\theta_{Ver-Final})+x_3 \qquad (22)$$

$$y_4=L_0/\tan(\theta_{Ver-Final})\times\cos(\theta_{Hor-Final})+y_3 \qquad (23)$$

Alternatively, the apparatus 100 for detecting a fixation point can further comprise a cursor moving unit 112. The cursor moving unit 112 determines whether it is needed to move the cursor. In case of need, the cursor is moved to the fixation point. Otherwise, the cursor is not moved. Preferably, affected by calculation accuracy and other factors, certain deviation may exist between the actual fixation point and the calculated fixation point D. In order to allow this deviation, the concept of fixation area is introduced. This area refers to a circular area on the screen with point D (the calculated fixation point) as the center and a predefined length G as the radius. Thus, when a new fixation point D is obtained, if the fixation point is located beyond the displayable scope of the screen, the cursor is not moved. Additionally, as long as the distance between the current cursor and point D is less than the predefined value G, the cursor will not be moved. Otherwise, the cursor is moved to the fixation point D.

Alternatively, the apparatus 100 for detecting a fixation point can further comprise an auxiliary unit 110. The user can perform operations at the cursor location through the auxiliary unit, for example, one or more of a mouse, a keyboard, a touchpad, a handle, and a remote controller. For example, the user can use the mouse to perform single click or double click operation or use a handle or remote controller to perform various kinds of key operations.

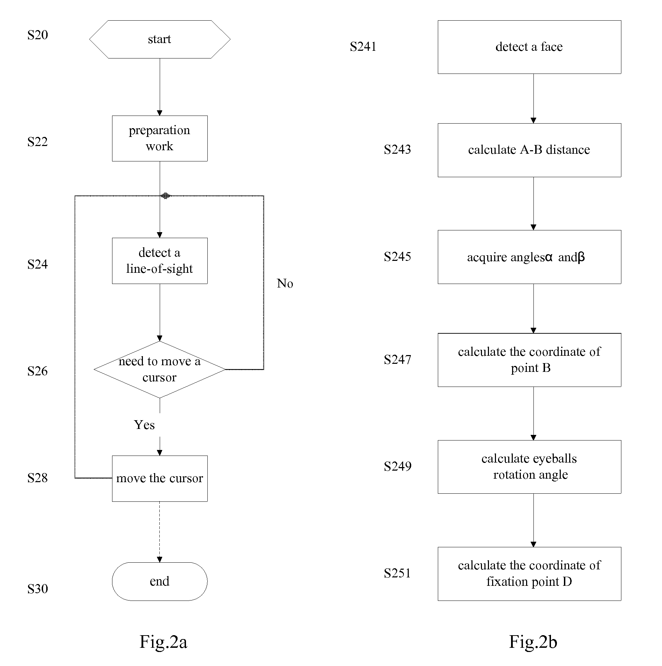

In the below, various steps of a method of detecting a fixation point according to the embodiments of the present invention will be described with reference to Figs 2a and 2b.

As illustrated in Fig. 2a, the method starts from step S20.

At step S22, preparation work is performed. The preparation work comprises: reference face images are collected by the camera, which are obtained with distances Do and $D_1$ in this embodiment. The reference face images are critical to the face detection/identification of the user. After determining the reference face images, the distance between two central points of the two pupils is obtained as the reference pupil distance $P_0$ and $P_1$. Next, the above mentioned eyeball direction table and projection round radius-cone vertex angle table are constructed. Or, if the two tables have been constructed and stored in the reference table storing unit, then they are just directly read. Finally, the location of the camera is located, i.e., the coordinate $(x_1, y_1, 0)$ of point A.

At step S24, fixation point detection is performed. Fig. 2b illustrates specific steps of detecting the fixation point. Specifically, at step S241, the face, pupil outlines, and pupil distance P of the user are detected. At step S243, AB distance L is calculated based on the pupil distance P, reference pupil distances $P_0$ and $P_1$. At step S245, angles $\alpha$ and $\beta$ are obtained. At step S247, the coordinate of point B is calculated. Afterwards, at step S249, rotation angle of the eyeballs are calculated. As above mentioned, the outline of the pupil of the left eye is detected based on the image captured by the camera, and the most suited outline is looked up in the eyeball direction table as above mentioned. In combination with the face direction, the vertical rotation angle $^\theta$Ver-L of the eyeball relative to the Yb axis and the horizontal rotation angle $^\theta$Hor-L relative to the Xb axis are obtained. $^\theta$Ve-R and $^\theta$Hor-R of the right eye can also be obtained in accordance with the same steps. Then, the line-of-sight direction of the user is calculated. Finally, at step S251, the coordinate $(x_4, y_4, 0)$ of the fixation point D on the screen 108 is calculated based on the calculated line-of-sight direction of the user.

After the step S24 of detecting the fixation point is implemented, with reference to Fig. 2a, alternatively, whether it is needed to move the cursor is determined at step S26. In case of need, then at step S28, the cursor is moved to the fixation point. Otherwise, the cursor is not moved. Afterwards, the method flow can return to step S24 to circularly perform detection of the fixation point. In case of terminating the method, then the method ends at step S30.

To sum up, the present invention provides a method and an apparatus for detecting a fixation point based on face detection and image measurement. Through detecting a face direction and eyeballs direction of a user and calculating the fixation point of the user on the screen, the cursor can be moved to the area. As required by calculation accuracy, a possible fixation area can be calculated, into which the cursor is moved, and then, the user manually moves the cursor to the expected accurate location, such that the actual movement distance of the user is dramatically shortened, and meanwhile, the calculation charge of the apparatus for detecting a fixation point is alleviated. The above solution can intentionally be implemented by setting a greater predefined radius G based on the actual apparatus accuracy.

Additionally, the detection method and apparatus according to the present invention can also be applied to a multi-screen computer having multiple screens around a user. The specific implementation is that: when there are multiple screens, the orientations of respective screens and their angle relations with the plane where a camera is located. When detecting a line-of-sight of the user, by utilizing the above principle of the present invention and calculating the intersection point of the line-of-sight extension line and relevant plane, the fixation point is finally obtained.

Although the present invention has been illustrated with reference to the preferred embodiments hereof, those skilled in the art would understand, without departing from the spirit and scope of the present invention, various amendments, replacements and alterations can be performed to the present invention. Thus, the present invention should not be defined by the aforementioned embodiments, but should be defined by the appended claims and their equivalents.

**Claims**

1. An apparatus for detecting a fixation point, which is used for calculating a fixation point of a user on a screen, comprising:

a camera for capturing a face image of the user;

a reference table acquiring unit for acquiring a reference table comprising relations between reference face images and line-of-sight directions of the user; and

a calculating unit for performing image measurement based on the face image of the user captured by the camera and looking up the reference table in the reference table acquiring unit, so as to calculate the fixation point of the user on the screen.

2. The apparatus for detecting a fixation point as claim 1, wherein the reference table acquiring unit comprises at least one of:

a reference table constructing unit for constructing the reference table based on at least one reference face image of the user captured by the camera; and

a reference table storing unit that stores the reference table which has already been constructed.

3. The apparatus for detecting a fixation point as claim 1, wherein the calculating unit comprises:

a line-of-sight direction calculating unit for measuring a distance between a middle point of two pupils of the user in the face image of the user and the camera based on a location of the camera, and calculating the line-of-sight direction of the user through looking up the reference table; and

a fixation point calculating unit for calculating the fixation point of the user on the screen based on the location of the camera, the distance between the middle point of two pupils of the user and the camera, and the line-of-sight direction of the user.

4. The apparatus for detecting a fixation point as claim 1, further comprises: a cursor moving unit, wherein, after the fixation point is calculated, if the fixation point is located within the screen, then the cursor moving unit moves the cursor on the screen to the fixation point.

5. The apparatus for detecting a fixation point as claim 1, wherein, if the distance between the fixation point and the current cursor is less than a predefined value, then the cursor moving unit does not move the cursor.

6. The apparatus for detecting a fixation point as claim 4 or 5, further comprising: an auxiliary unit for performing operation at the cursor location.

7. The apparatus for detecting a fixation point as claim 6, wherein the auxiliary unit comprises at least one of a mouse, a keyboard, a touchpad, a handle, and a remote controller.

8. A method of detecting a fixation point, for calculating a fixation point of a user on a screen, which comprises steps of:

a reference table acquiring step of acquiring a reference table comprising relations between reference face images and line-of-sight directions of the user;

a fixation point calculating step of using a camera to capture a face image of the user, performing image measurement and looking up the reference table, so as to calculate the fixation point of the user on the screen.

9. The method as claim 8, wherein the reference table acquiring step comprises:

using the camera to acquire at least one reference face image of the user to construct the reference table comprising relations between the reference face images and the line-of-sight directions of the user; or

directly obtaining the reference table which has already been constructed.

10. The method as claim 8, wherein the fixation point calculating step comprises:

measuring a distance between a middle point of two pupils of the user in the face image of the user and the camera based on a location of the camera, and calculating the line-of-sight direction of the user through looking up the reference table; and

calculating the fixation point of the user on the screen based on the location of the camera, the distance between the middle point of two pupils of the user and the camera, and the line-of sight direction of the user.

11. The method as claim 8, further comprising: after calculating the fixation point, if the fixation point is within the screen, the cursor on the screen is moved to the fixation point.

12. The method as claim 11, wherein if the distance between the fixation point and the current cursor is less than a predefined value, the cursor is not moved.

13. The method as claim 12, wherein the predefined value is set as required.

14. A multi-screen computer having multiple screens around a user, wherein the multi-screen computer comprises the apparatus for detecting a fixation point as any one of claims 1-7.

Apparatus 100 for Detecting a Fixation Point

```
                          ╭───────╮
                          │camera │──── 102
                          ╰───────╯

         ┌─ 104                    ┌─ 106
  ┌─────────────────┐    ┌──────────────────────┐
  │ reference table │    │   calculating unit    │        ┌─ 110
  │ acquiring unit  │    │            ┌─ 1062     │  ┌──────────────┐
  │      ┌─ 1042    │    │  ┌──────────────────┐  │◄─│ auxiliary unit│
  │ ┌──────────────┐│    │  │  line-of-sight   │  │  └──────────────┘
  │ │reference table││───┼─►│    direction     │  │          │
  │ │constructing   ││    │  │ calculating unit │  │          ▼  ┌─ 112
  │ │unit          ││    │  └──────────────────┘  │  ┌──────────────┐
  │ └──────────────┘│    │            ┌─ 1064     │  │cursor moving │
  │      ┌─ 1044    │    │  ┌──────────────────┐  │  │    unit      │
  │ ┌──────────────┐│    │  │  fixation point  │──┼─►└──────────────┘
  │ │reference table││    │  │ calculating unit │  │          │
  │ │storing unit  ││    │  └──────────────────┘  │          │
  │ └──────────────┘│    └──────────────────────┘          │
  └─────────────────┘              │                         │
                                   │  ┌─ 108                  │
                      ┌────────────▼──────────────────────────▼─┐
                      │                screen                   │
                      └─────────────────────────────────────────┘
```

Fig.1

S20    start

S22    preparation
       work

S24    detect a
       line-of-sight

       No

S26    need to move a
       cursor

       Yes

S28    move the cursor

S30    end

Fig.2a

S241    detect a face

S243    calculate A-B distance

S245    acquire angles α and β

S247    calculate the coordinate of
        point B

S249    calculate eyeballs
        rotation angle

S251    calculate the coordinate of
        fixation point D

Fig.2b

Fig. 3

Fig. 4

Fig. 5a

| 01 | 02 | 03 |
|----|----|----|
| 11 | 12 | 13 |
| 21 | 22 | 23 |

Fig. 5b

Fig. 6a

Fig. 6b

Image captured by the camera

Fig. 7

Fig. 8

Fig. 9

| index | $\theta_{Ver}$ | $\theta_{Hor}$ | substantial face direction | pupil outline shape |
|---|---|---|---|---|
| $I_M$ | 90.0 | 0.0 | 11 | |
| $I_N$ | 90.0 | 90.0 | 12 | |
| $I_Q$ | 0.0 | 90.0 | 22 | |
| $I_{Q'}$ | 180.0 | 90.0 | 02 | |
| $I_0$ | 80.0 | 30.0 | 21 | |

Fig. 10

| index | projection round radius (pixel) | cone vertex angle |
|---|---|---|
| 1 | 0 | 0.0 |
| 2 | 20 | 2.0 |
| 3 | 40 | 4.0 |
| 4 | 60 | 6.0 |
| 5 | 80 | 8.0 |
| 6 | 100 | 10.0 |
| 7 | 120 | 12.0 |
| 8 | 140 | 14.0 |
| 9 | 160 | 16.0 |
| 10 | 180 | 18.0 |
| 40 | 200 | 20.0 |

Fig. 11

# INTERNATIONAL SEARCH REPORT

| | International application No. |
|---|---|
| | PCT/CN2009/001105 |

### A. CLASSIFICATION OF SUBJECT MATTER

See the extra sheet
According to International Patent Classification (IPC) or to both national classification and IPC

### B. FIELDS SEARCHED

Minimum documentation searched (classification system followed by classification symbols)

IPC: G06F; G06K; A61B3/-

Documentation searched other than minimum documentation to the extent that such documents are included in the fields searched

Electronic data base consulted during the international search (name of data base and, where practicable, search terms used)

CNPAT;CNKI;WPI;EPODOC: sight, gaz+, view+, visual, line, axis, detect+, measurement, direction?, face?, head, pupil?, eyeball?, cent??, attitude+, screen

### C. DOCUMENTS CONSIDERED TO BE RELEVANT

| Category* | Citation of document, with indication, where appropriate, of the relevant passages | Relevant to claim No. |
|---|---|---|
| X | CN101419672A(COMPUTING TECHNOLOGY RES. INST. CHINESE AC.) 29Apr.2009(29.04.2009) Description page 4 line 17- page 7 line 29, figures 2-5 | 1,2,8,9,14 |
| Y | | 3-7,10-13 |
| Y | CN101489467A(MATSUSHITA ELECTRIC IND. CO., LTD.) 22 Jul.2009 (22.07.2009) Description page 5 paragraph 5 line 5 – page 6 line 5 | 3,10 |
| Y | CN101311882A(HUAWEI TECHNOLOGIES CO.,LTD.) 26 Nov.2008 (26.11.2008) Description page 1 paragraph 4, page 7 line 23-page 8 paragraphs 1-3 | 4-7,11-13 |
| A | CN101326546A(MATSUSHITA ELECTRIC IND. CO., LTD.) 17 Dec.2008 (17.12.2008) The whole document | 1-14 |

☒ Further documents are listed in the continuation of Box C.　　☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim (S) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 30 Nov.2009(30.11.2009) | **31 Dec. 2009 (31.12.2009)** |

| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br>　　　　YUE,Yongjuan<br>Telephone No. (86-10)62414109 |
|---|---|

Form PCT/ISA /210 (second sheet) (July 2009)

## INTERNATIONAL SEARCH REPORT

International application No.

PCT/CN2009/001105

C (Continuation).     DOCUMENTS CONSIDERED TO BE RELEVANT

| Category* | Citation of document, with indication, where appropriate, of the relevant passages | Relevant to claim No. |
|---|---|---|
| A | US2005/0200806A1(KNAAN,Dotan et al.) 15 Sep.2005(15.09.2005) The whole document | 1-14 |
| A | JP2009-42956A (HITACHI LTD. et al.) 26 Feb.2009(26.02.2009) The whole document | 1-14 |
| A | DE19819961A1(KUKULENZ D.) 11 Nov.1999(11.11.1999) The whole document | 1-14 |

Form PCT/ISA /210 (continuation of second sheet ) (July 2009)

## INTERNATIONAL SEARCH REPORT
### Information on patent family members

| | International application No. |
|---|---|
| | PCT/CN2009/001105 |

| Patent Documents referred in the Report | Publication Date | Patent Family | Publication Date |
|---|---|---|---|
| CN101419672A | 29.04.2009 | None | |
| CN101489467A | 22.07.2009 | WO2008007781A1 | 17.01.2008 |
| | | EP2042079A1 | 01.04.2009 |
| CN101311882A | 26.11.2008 | None | |
| CN101326546A | 17.12.2008 | WO2007074842A1 | 05.07.2007 |
| | | JP2007200298A | 09.08.2007 |
| | | EP1968006A1 | 10.09.2008 |
| US2005200806A1 | 15.09.2005 | EP1580649A1 | 28.09.2005 |
| | | JP2005253778A | 22.09.2005 |
| | | US7246904B2 | 24.07.2007 |
| JP2009042956A | 26.02.2009 | None | |
| DE19819961A1 | 11.11.1999 | None | |

Form PCT/ISA /210 (patent family annex) (July 2009)

## INTERNATIONAL SEARCH REPORT

International application No.

PCT/CN2009/001105

**CLASSIFICATION OF SUBJECT MATTER**

G06F3/01 (2006.01) i
G06K9/00 (2006.01) i
A61B3/113 (2006.01) i
G06F3/033 (2006.01) n
G06K9/20 (2006.01) n